# EUROPEAN PATENT SPECIFICATION

(11) **EP 3 258 905 B1**
(45) Date of publication and mention of the grant of the patent: **15.05.2019**
(21) Application number: 16704615.0
(22) Date of filing: 15.02.2016
(51) Int. Cl.: A61K 8/25, A61K 8/29, A61Q 11/00, A61K 8/02

(54) **ORAL CARE COMPOSITION**
MUNDPFLEGEMITTEL
COMPOSITIONS DE PROTECTION ORALE

(30) Priority: 16.02.2015 EP 15155225
(43) Date of publication of application: 27.12.2017
(73) Proprietor: Unilever PLC, London, Greater London EC4Y 0DY (GB); Unilever N.V., 3013 AL Rotterdam (NL)
(72) Inventor: GREEN, Alison, Katharine, Wirral Merseyside CH63 3JW (GB); LIMER, Adam, John, Wirral Merseyside CH63 3JW (GB); PHILPOTTS, Carole, Jane, Wirral Merseyside CH63 3JW (GB)
(74) Representative: Tansley, Sally Elizabeth
(86) International application number: PCT/EP2016/053109
(87) International publication number: WO 2016/131745

(56) References cited:
- WO-A1-2008/068149
- WO-A1-2011/109919
- GB-A- 2 144 631
- KR-A- 20040 021 954
- US-B1- 6 343 400
- DATABASE WPI Week 200382 Thomson Scientific, London, GB; AN 2003-883167 XP002743155, -& JP 2003 221322 A (SUGIYAMA Y) 5 August 2003 (2003-08-05)
- DATABASE WPI Week 201460 Thomson Scientific, London, GB; AN 2014-R18946 XP002743156, -& CN 103 893 032 A (DALIAN LINQIAO SCI & TECHNOLOGY CO LTD) 2 July 2014 (2014-07-02)
- DATABASE WPI Week 201504 Thomson Scientific, London, GB; AN 2014-R18967 XP002743157, -& CN 103 893 021 A (DALIAN LINQIAO SCI & TECHNOLOGY CO LTD) 2 July 2014 (2014-07-02)
- DATABASE WPI Week 201429 Thomson Scientific, London, GB; AN 2014-H52288 XP002743158, -& CN 103 622 847 A (HESHUANG TECHNOLOGY CO LTD HECHUAN DISTR) 12 March 2014 (2014-03-12)

## Description

The present invention relates to oral care compositions, in particular the present invention relates to oral compositions that whiten the teeth.

### Field of the Invention

There are several commonly used ways of whitening the teeth. Brushing with a dentifrice, whether a toothpaste, gel, cream, or powder, has some effect in whitening teeth due to the abrasive action of the dentifrice on the teeth. However a balance is needed between the damage caused by the use of abrasives and the whitening effect.

A second method of whitening teeth comprises brushing them with a dentifrice comprising hydrogen peroxide. Such systems are disclosed in US6036493, use of such products leads to temporary tooth hypersensitivity and potential gingival irritation.

US7261558 and US4910014 disclose methods of delivering bleaching agents in the oral cavity but these suffer from poor substantivity and hence short treatment times.

UV light emitting sources have been cited as lightening teeth as described in US7261558 and US5306143.

WO2008/068149 oral care products that remineralise and whiten the teeth. The products comprise mesoporous calcium silicate biomaterial (MCBS) dispersed in a polymeric material matrix.

There remains the need for enhanced whitening of the teeth without the harshness of abrasive or peroxide based whitening systems.

### Description of the Invention

The present invention provides an oral care product suitable for whitening the teeth the oral care composition comprising uncoated particles of titanium dioxide having a particle size of less than 90nm and a deposition means for depositing the titanium dioxide
particles onto the surface of a tooth in which the deposition means comprises calcium silicate.

The invention further relates to a method of whitening teeth comprising the step of application to the teeth of the product described above.

Further described is a kit for lightening the teeth comprising:
i) the product described above; and
ii) a source of UV light

### Detailed Description of the Invention

In the context of the present invention the term particle size relates to weight mean particle size d(50). The weight mean particle size of the silica may suitably be determined using a Malvern Mastersizer® model S, with a 300 RF lens and MS 17 sample presentation unit. This instrument, made by Malvern Instruments, Malvern, Worcestershire, uses the principle of Fraunhofer diffraction, utilising a low power He/Ne laser.

In the context of the present invention the term uncoated titanium dioxide means that the titanium dioxide has not been reacted or coated with another material.

Compositions of the invention comprise uncoated titanium dioxide having a weight mean particle (d50) size of less than 90nm, preferably the titanium dioxide has a particle size of less than 50 nm, most preferably a particle size less than 40 nm. It is especially preferred the titanium dioxide has a particle size (d50) less than 30nm. It is preferred if the titanium dioxide has a particle size greater than 2nm, more preferably greater than to 5nm, most preferably greater than 10nm.

Preferably the level of the level of uncoated titanium dioxide is from 0.1 to 20 wt% of the total composition, more preferably from 0.25 to 15 wt%, most preferably 0.5 wt% to 7 wt% of the total composition.

Products according to the invention comprise a deposition means. It is preferable if the deposition means comprises a mixture of a calcium source and a phosphate source which, when delivered to the teeth results in the *in situ* generation of hydroxyapatite on teeth. This calcium source/phosphate source deposition means has the additional advantage that it remineralises (deposits hydroxyapatite) the teeth, in that it can aid repair to damaged enamel damage.

The source of calcium ions is calcium silicate.

The total amount of calcium source(s) (e.g. calcium silicate) in the composition of the invention typically ranges from 1 to 30%, preferably from 5 to 20% by total weight based on the total weight of the oral care composition.

Illustrative examples of the types of phosphate source that may be used in this context include, for example, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the phosphate source is a trisodium phosphate or sodium dihydrogen phosphate or particularly preferred a mixture of trisodium phosphate and sodium dihydrogen phosphate.

The amount of phosphate source(s) (e.g. trisodium phosphate and sodium dihydrogen phosphate) in the product according to the invention typically ranges from 0.001 wt% to 7.0 wt%, more preferably 0.005 to 3.5 wt%, most preferably 0.01 wt% to 1.0 wt% of the total product.

Mixtures of any of the above described materials may also be used.

### Dual phase composition

It is preferred if the product comprises two phases; the first phase being non-aqueous and the second phase being aqueous. The two phases of the product may be packaged separately or may be part of a dual phase composition.

It is preferable if the phosphate source and titanium dioxide are present in different phases. It is particularly advantageous if greater than 50 wt% of the phosphate source is in the aqueous phase, particularly preferred if 95 wt% of the phosphate source is in the aqueous phase.

It is preferred if the calcium source is in the non-aqueous phase.

In the event that the product comprises a composition having a single phase it is advantageous if the composition is non-aqueous.

In the event that the composition is a dual phase composition it is preferable if the first composition comprises a non-aqueous liquid continuous phase comprising the untreated titanium dioxide and calcium source.

The amount of calcium silicate in the first composition according to the invention typically ranges from 1 to 30%, preferably from 5 to 20% by total weight calcium source based on the total weight of the first composition.

The first composition according to the invention is non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% by weight based on the total weight of the first composition.

The liquid continuous phase of the first composition of the invention preferably comprises a thickening agent. The thickening agent helps to impart a desirable viscosity profile and desirable flow characteristics to the composition.

Suitable thickening agents for use in the first non-aqueous composition according to the invention are those which are operable in non-aqueous systems. Examples of such materials include organic macromolecules which do not necessarily require hydration with water in order to build viscosity, but can also build viscosity by alternative mechanisms such as by hydrogen bonding in the presence of hydroxyl donors such as polyols. Carboxyvinyl polymers have been found to be useful in this context. Preferred carboxyvinyl polymers for use in the invention have a molecular weight of at least about 750,000, more preferably at least about 1,250,000, most preferably at least about 3,000,000 g/mol. A suitable chemical class of such carboxyvinyl polymers for use in the invention includes crosslinked polymers having a polymer backbone derived from acrylic acid, substituted acrylic acid, or salts or esters thereof, in which the crosslinking agent contains two or more carbon-carbon double bonds and is derived from a polyhydric alcohol. Specific examples of such materials are homopolymers of acrylic acid cross-linked with allyl ethers of sucrose or pentaerythritol and homopolymers of acrylic acid cross-linked with divinyl glycol. An example of a suitable carboxyvinyl polymer for use in the compositions of the present invention is a homopolymer of acrylic acid crosslinked with allyl ethers of pentaerythritol, which is slightly pre-neutralised (1 to 3%) by potassium salt. This pre-neutralisation is done in order to precipitate polyacrylic acid in the presence of the polymerisation solvent. Such a material is commercially available, for example, as CARBOPOL® 974P NF Polymer, ex Lubrizol Advanced Materials, Inc. For ease of processing, the most preferred carboxyvinyl polymer for use in the compositions of the present invention is a homopolymer of acrylic acid crosslinked with pentaerythritol triallylether. Such a material is commercially available, for example, as SYNTHALEN® KP, ex 3V Sigma. Mixtures of any of the above described materials may also be used.

The level of thickening agent will depend on the particular type chosen, but generally ranges from 0.01 to 10% by total weight thickener based on the total weight of the first composition. When the thickening agent is a carboxyvinyl polymer (as described above), the amount of carboxyvinyl polymer in the first composition suitably ranges from 0.01 to 5%, preferably from 0.02 to 1%, more preferably from 0.05 to 0.25% by total weight carboxyvinyl polymer based on the total weight of the first composition.

The liquid continuous phase of the first non-aqueous composition of the invention preferably comprises a humectant. The humectant helps to keep the composition from hardening or crystallizing upon exposure to air. It also helps to give the composition a moist feel to the mouth, and may in some cases impart a desirable sweetness.

Preferred humectants for use in the invention include organic polyols having 3 or more hydroxyl groups in the molecule (hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of humectant will depend on the particular type chosen, but generally ranges from about 20 to 90% by weight based on the total weight of the first composition. When the humectant is one or more organic polyols (as described above), the amount of organic polyol suitably ranges from 35 to 75%, more preferably from 45 to 70% by total weight organic polyol based on the total weight of the composition. Most preferably the first composition of the invention is organic polyol-based.

In the context of the present invention, the term "organic polyol-based" means that the composition is not oil-based or water-based, but instead, organic polyols (as defined above) are a principal component in the composition. By "principal component" is meant that the organic polyols (as defined above) when taken together, make up a higher portion of the first composition's weight than any other compound. Ideally the first composition of the invention is glycerol-based (i.e. glycerol makes up a higher portion of the composition's weight than any other compound) and contains from 45 to 70% by weight glycerol based on the total weight of the first composition.

The first composition of the invention is typically in the form of an extrudable semi-solid such as a cream, paste or gel (or mixture thereof).

The viscosity of the first composition of the invention generally ranges from 10,000 to 100,000 mPa.s (10,000 to 100,000 cps), and preferably ranges from 30,000 to 60,000 mPa.s (30,000 to 60,000 cps), (when measured at 25°C on a Brookfield viscometer).

The pH of the first composition of the invention generally ranges from 7 to 10.5, and preferably ranges from 8 to 9, when measured at 25° C using conventional pH sensitive electrodes.

The first composition according to the invention may optionally include further ingredients to enhance performance and/or consumer acceptability.

The second composition having an aqueous liquid continuous phase preferably comprises the phosphate source and preferably a thickening agent and/or a humectants. The level of water in the second composition is at least 30% by weight water (by weight based on the total weight of the second composition).

Suitable thickening agents for use in the second composition according to the invention are those which are operable in aqueous systems. Examples of such materials include cellulose based gums.

A preferred class of cellulose based gum for use in the second composition according to the invention are cellulose ethers.

Specific examples of such materials include: carboxyalkyl cellulose ethers such as carboxyethyl cellulose and carboxymethyl cellulose (CMC); mixed ethers such as carboxyalkyl hydroxyalkyl ethers, for example carboxymethyl hydroxyethyl cellulose (CMHEC); hydroxyalkyl celluloses such as hydroxyethyl cellulose and hydroxypropyl cellulose; alkylhydroxyalkyl celluloses such as methylhydroxypropyl cellulose; alkyl celluloses such as methyl cellulose, ethyl cellulose, and propyl cellulose; alkylcarboxyalkyl celluloses such as ethylcarboxymethyl cellulose; alkylalkyl celluloses such as methylethyl cellulose; and hydroxyalkylalkyl celluloses such as hydroxypropylmethyl cellulose. The above cellulose ethers are generally available commercially in various grades. The carboxy-substituted cellulose ethers are available as the alkali metal salt, usually the sodium salt (SCMC). SCMC is commercially available in various degrees of carboxylate substitution ranging from about 0.3 up to the maximum degree of substitution of 3.0. Preferred sodium carboxymethyl celluloses (SCMCs) include those with a degree of substitution of from 0.65 to 0.95, more preferably from 0.85 to 0.95, and a viscosity ranging from 250 to 10000 mPa.s, more preferably from 1,000 to 6,000 mPa.s (when measured as a 1% solution in distilled water at 25°C on a Brookfield viscometer).

The level of thickening agent will depend on the particular type chosen, but generally ranges from 0.01 to 10% by total weight thickener based on the total weight of the second composition. When the thickening agent is a cellulose ether (as described above), the amount of cellulose ether in the second composition suitably ranges from 0.01 to 5%, preferably from 0.02 to 1%, more preferably from 0.5 to 1.5% by total weight cellulose ether based on the total weight of the second composition.

Suitable humectants for use in the second composition according to the invention include organic polyols having 3 or more hydroxyl groups in the molecule (as described above in relation to the first composition and hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of humectant will depend on the particular type chosen, but generally ranges from about 20 to 90% by weight based on the total weight of the second composition. When the humectant is one or more organic polyols (as described above), the amount of organic polyol suitably ranges from 30 to 65%, more preferably from 45 to 50% by total weight organic polyol based on the total weight of the second composition.

The second composition according to the invention comprises at least 30% by weight water (by weight based on the total weight of the second composition). Preferably the level of water ranges from 50 to 65%, more preferably from 50 to 55% by weight based on the total weight of the second composition.

The second composition of the invention is typically in the form of an extrudable semi-solid such as a cream, paste or gel (or mixture thereof).

The viscosity of the second composition of the invention generally ranges from 1,000 to 30,000 mPa.s (1,000 to 30,000 cps), and preferably ranges from 10,000 to 20,000 mPa.s (10,000 to 20,000 cps), when measured at 25°C on a Brookfield viscometer.

The pH of the second composition of the invention generally ranges from 7 to 9, and preferably ranges from 7.5 to 8.5, when measured at 25° C using conventional pH sensitive electrodes.

The second composition according to the invention may optionally include further ingredients to enhance performance and/or consumer acceptability.

In use the first and second compositions are preferably adapted to be admixed and applied to the surface of the teeth for sustained contact.

### Further Optional ingredients

Products according to the invention may include other optional ingredients. Examples of such ingredients include abrasive amorphous silica particles which have a weight mean particle size (d50) ranging from 3 to 15 microns.

Preferred abrasive amorphous silica particles for use in the invention have a weight mean particle size in the range 3 to 6 microns.

Preferably, the abrasive amorphous silica particles employed are precipitated silica. Suitable precipitated silicas for use as abrasive amorphous silica particles in the invention are commercially available and include those marketed by PQ Corporation under the trade names SORBOSIL® AC 43, AC77, AC35 and SORBOSIL® AC 33.

Mixtures of any of the above described materials may also be used.

The level of abrasive amorphous silica particles (as defined above) generally ranges from 0.05 to 5%, preferably from 0.1 to 3%, more preferably from 0.2 to 0.8%, by total weight abrasive amorphous silica particles (as defined above) based on the phase in which they are present.

Preferably the abrasive amorphous silica is present in the non aqueous phase/composition.

In one preferred embodiment the product is a serum and comprises less than 0.1 wt% of the total product of abrasive, preferably less than 0.05 wt%. Serums are more effective at delivering the whitening agent then conventional toothpastes comprising abrasive silicas.

Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Mixtures of any of the above described materials may also be used.

The level of surfactant generally ranges from 0.2 to 5% by total weight surfactant based on the total weight of the composition/phase in which it is present.

Preferably surfactants are present in the non aqueous phase/composition.

The composition may also contain one or more liquid polyethylene glycols having a melting point below 25°C. Preferably the melting point ranges from -50 to 22°C, more preferably from -15 to 8° C, most preferably from 4 to 8°C.

The liquid polyethylene glycol(s) helps to make processing of the composition easier, especially at high shear, by reducing the overall viscosity of the liquid continuous phase.

Preferred liquid polyethylene glycols have an average value of n in the general formula H(OCH2CH2)n OH (as described above) ranging from about 4 to 12, more preferably about 6 to 8, most preferably about 8. The average molecular weight suitably ranges from about 190 to 630, more preferably from about 285 to 420, most preferably from about 380 to 420 g/mol. Suitable commercially available materials include for example PEG 400 (ex BDH Chemicals). Mixtures of any of the above described materials may also be used.

The amount of liquid polyethylene glycol (as defined above) suitably ranges from 1 to 20%, preferably from 5 to 15%, more preferably from 8 to 12% by total weight liquid polyethylene glycol (as defined above) based on the total composition/phase in which they are present.

Preferably the liquid polyethylene glycol is present in the non aqueous phase

The product may also include one or more solid particulate polyethylene glycols, preferably in the non-aqueous phase/composition having a melting point of 25°C or above. Preferably the melting point ranges from 35 to 65°C, more preferably from 55 to 60° C.

Polyethylene glycols have the general formula H(OCH2CH2)n OH, where n is the number of repeating oxyethylene units. Commercially available polyethylene glycols are usually not uniform chemical compounds, but instead consist of a distribution of similar polymer members of the homologous polyethylene glycol series, defined by average values of n and molecular weight. The melting point generally increases with increasing average values of n and molecular weight. Suitable solid polyethylene glycols have an average value of n in the above general formula ranging from about 20 to 220, preferably from about 40 to 150, more preferably from about 32 to 90, most preferably from about 60 to 75. The average molecular weight suitably ranges from about 950 to 11,250, preferably from about 1800 to 6600, more preferably from about 1400 to 4400, most preferably from about 2700 to 3700 g/mol. Suitable commercially available materials include for example Polyglykol® 3000 (ex Clariant). Mixtures of any of the above described materials may also be used.

The amount of solid polyethylene glycol (as defined above) suitably ranges from 0.1 to 5%, preferably from 0.5 to 3%, more preferably from 1 to 2.5% by total weight solid polyethylene glycol (as defined above) based on the total weight of the composition in which they are present.

The particles of solid polyethylene glycol(s) help to provide an optimised microstructure for the composition, particularly when the composition is prepared by a process route involving carefully controlled heating and cooling to form crystals of the solid polyethylene glycol.

Also suitable for addition to the products of the invention are fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.
further optional ingredients customary in the art such as buffers, flavouring agents, sweetening agents, colouring agents, opacifying agents, preservatives and the like.

### Product Form and Packaging

If the composition of the invention is present as a dual composition, the second composition is stored separately from the first composition prior to usage of the product in a manner to avoid contact between the water in the second composition and the calcium source in the first composition.

In packaging the oral care product of the invention, any convenient means for effecting the separation of the first composition from the second composition before use may be used. For example, a single container may be compartmentalized so that the first composition and the second composition are housed in separate compartments and are dispensed synchronously as a ribbon, and admixed prior to application on the teeth. Alternatively, the first composition and the second composition may be housed in physically separate containers (e.g. tubes) from which they are dispensed for admixture just prior to use.

It is preferred if the product is substantially free of peroxide. In the context of the present invention substantially free means comprises less than 0.05 wt% of the total product, more preferably less than 0.005 wt% of the total product.

### Mode of Use

Compositions of the invention are to be applied to the teeth. In the event that the product comprises two, the first and second compositions are adapted to be admixed and applied to the surface of the teeth preferably for sustained contact.

In the context of the present invention sustained contact means the product is left on the teeth for 1 to 10 minutes, preferably about 2 to 5 minutes before being removed, before being removed by brushing or rinsing.

For example, separate ribbons of the first and second compositions respectively may be extruded sequentially onto the bristles of a toothbrush, which is then agitated against the surface of the teeth.

In a particularly efficacious whitening treatment the teeth are subjected to a UV light source after treatment. Preferably the UV light is shone on the teeth for 1 to 15 minutes.

The invention will now be illustrated by the following Examples. Examples of the invention will be illustrated by a number, comparative examples will be illustrated by a letter.

### Examples

A formulation was prepared by combining the following materials:

| | **Example A** | **Example 1** | **Example B** | **Example C** |
|---|---|---|---|---|
| Glycerol | 8.4g | 8.4g | 8.4g | 9.6g |
| Titanium Dioxide (Hombitan FF-Pharma) | 2.4g | | | |
| Titanium Dioxide (Aerosil P25) | | 2.4g | 2.4g | 2.4g |
| Calcium Silicate (Sorbosil CA40) | 1.2g | 1.2g | | |
| Hydroxyapatite | | | 1.2g | |

Titanium dioxide Hombitan FF-Pharma particle size 170nm
Titanium dioxide Aerosil P25 particle size 21nm
A product slurry was prepared by mixing 1.5g formulation with 1g of 0.125wt% phosphate solution. Bovine block samples (n=4) were incubate in slurry for 30 minutes with gentle agitation and then incubated in simulated oral fluid for 6 hours. Application process was completed a total of 3 times for each sample. Excess product was removed from sample edges and surface after each application by hand water brushing the blocks with a medium bristle toothbrush for 5 seconds. The sample was immersed in deionised water and exposed to UV light for 1.5 hours (2x 8w @ 365nm).

Colour was measured via chromameter at baseline, after slurry application and after UV light exposure. L*a*b* colour parameters were converted to WIO whiteness indices to allow comparison between samples.

Colour change is expressed as ΔWIO1 = WIO(slurry application)-WIO(baseline) and ΔWIO2 = WIO(UV Light Exposure)-WIO(baseline).

| | **ΔWIO1** | **ΔWIO2** |
|---|---|---|
| Example A | 14.7 (± 3.4) | 16.1 (±3.2) |
| Example 1 | 14.4 (± 3.0) | 19.4 (±4.1) |
| Example B | -0.6 (±6.3) | -1.6 (±6.1) |
| Example C | 0.9 (±3.9) | 0.2 (±3.7) |

Example 1 of the invention shows enhanced photobleaching compared with the comparative Examples.

## Claims

1. An oral care product comprising uncoated particles of titanium dioxide having a weight mean particle size (d50) of less than 90nm and a means for depositing the titanium dioxide particles onto the surface of a tooth the deposition means comprising a calcium silicate.

2. An oral care product according to claim 1 in which the titanium dioxide has a weight mean particle size (d50) of less than 50 nm.

3. An oral care product according to any preceding claim in which the titanium dioxide has a weight mean particle size (d50) less than 30 nm.

4. An oral care product according to any preceding claim in which the titanium dioxide has a weight mean particle size (d50) from 5nm to 40nm.

5. An oral care product according to any preceding claim in which the deposition means comprises a phosphate source and a calcium source.

6. An oral care product according to claim 5 in which the phosphate source is trisodium phosphate, sodium dihydrogen phosphate or a mixture thereof.

7. An oral care product according to any preceding claim in which the calcium source is calcium silicate.

8. An oral care product according to any of preceding claim in which the level of uncoated titanium dioxide is from 0.25 to 15 wt% of the total composition.

9. An oral care product according to any preceding claim which comprises two phases; the first phase being non-aqueous such that water is present in an amount no greater the 5 wt% and the second phase being aqueous.

10. An oral care product according to claim 9 to in which at least 95 wt% of the phosphate source is in the aqueous phase.

11. An oral care product according to any one of claims 9 to 10 in which the calcium source is in the non-aqueous phase.

12. An oral care product according to any preceding claim that is substantially free (less than 0.05 wt% of the total product) of peroxide.

13. A method of whitening teeth comprising the following steps: applying to the teeth the product described in any preceding claims.

14. A kit for lightening the teeth comprising:
i) the product according to any one of claims 1 to 12; and
ii) a source of UV light

## Patentansprüche

1. Mundpflegeprodukt, umfassend unbeschichtete Teilchen von Titandioxid, die eine gewichtsmittlere Teilchengröße (d50) von weniger als 90 nm aufweisen, und ein Mittel zum Ablagern von Titandioxid-Teilchen auf der Oberfläche eines Zahns, wobei das Mittel zum Ablagern ein Calciumsilikat umfasst.

2. Mundpflegeprodukt nach Anspruch 1, in welchem das Titandioxid eine gewichtsmittlere Teilchengröße (d50) von weniger als 50 nm aufweist.

3. Mundpflegeprodukt nach irgendeinem vorhergehenden Anspruch, in welchem das Titandioxid eine gewichtsmittlere Teilchengröße (d50) von weniger als 30 nm aufweist.

4. Mundpflegeprodukt nach irgendeinem vorhergehenden Anspruch, in welchem das Titandioxid eine gewichtsmittlere Teilchengröße (d50) von 5 nm bis 40 nm aufweist.

5. Mundpflegeprodukt nach irgendeinem vorhergehenden Anspruch, in welchem das Mittel zum Ablagern eine Phosphat- und eine Calcium-Quelle umfasst.

6. Mundpflegeprodukt nach Anspruch 5, in welchem die Phosphat-Quelle Trinatriumphosphat, Natriumdihydrogenphosphat oder eine Mischung davon ist.

7. Mundpflegeprodukt nach irgendeinem vorhergehenden Anspruch, in welchem die Calcium-Quelle Calciumsilikat ist.

8. Mundpflegeprodukt nach irgendeinem vorhergehenden Anspruch, in welchem der Gehalt des unbeschichteten Titandioxids von 0,25 bis 15 Gew.-% der gesamten Zusammensetzung beträgt.

9. Mundpflegeprodukt nach irgendeinem vorhergehenden Anspruch, welches zwei Phasen umfasst, wobei die erste Phase nicht-wässrig ist, so dass Wasser in einer Menge von nicht größer als 5 Gew.-% vorliegt, und die zweite Phase wässrig ist.

10. Mundpflegeprodukt nach Anspruch 9, in welchem mindestens 95 Gew.-% der Phosphat-Quelle in der wässrigen Phase vorliegen.

11. Mundpflegeprodukt nach irgendeinem der Ansprüche 9 bis 10, in welchem die Calcium-Quelle in der nicht-wässrigen Phase vorliegt.

12. Mundpflegeprodukt nach irgendeinem vorhergehenden Anspruch, das im Wesentlichen frei (weniger als 0,05 Gew.-% des gesamten Produkts) von Peroxid ist.

13. Verfahren zum Aufhellen von Zähnen, umfassend die folgenden Schritte: Auftragen des Produktes, beschrieben in irgendeinem der vorhergehenden Ansprüche, auf die Zähne.

14. Kit zum Aufhellen von Zähnen, umfassend:
i) das Produkt nach irgendeinem der Ansprüche 1 bis 12
und
ii) eine Quelle von UV-Licht.

## Revendications

1. Produit pour le soin oral comprenant des particules non revêtues de dioxyde de titane présentant une taille moyenne de particule en masse (d50) inférieure à 90 nm et un moyen pour déposer les particules de dioxyde de titane sur la surface d'une dent, le moyen de dépôt comprenant un silicate de calcium.

2. Produit pour le soin oral selon la revendication 1, dans lequel le dioxyde de titane présente une taille moyenne de particule en masse (d50) inférieure à 50 nm.

3. Produit pour le soin oral selon l'une quelconque des revendications précédentes, dans lequel le dioxyde de titane présente une taille moyenne de particule en masse (d50) inférieure à 30 nm.

4. Produit pour le soin oral selon l'une quelconque des revendications précédentes, dans lequel le dioxyde de titane présente une taille moyenne de particule en masse (d50) de 5 nm à 40 nm.

5. Produit pour le soin oral selon l'une quelconque des revendications précédentes, dans lequel le moyen de dépôt comprend une source de phosphate et une source de calcium.

6. Produit pour le soin oral selon la revendication 5, dans lequel la source de phosphate est le phosphate de trisodium, le dihydrogéno-phosphate de sodium ou un mélange de ceux-ci.

7. Produit pour le soin oral selon l'une quelconque des revendications précédentes, dans lequel la source de calcium est le silicate de calcium.

8. Produit pour le soin oral selon l'une quelconque des revendications précédentes, dans lequel la teneur en dioxyde de titane non revêtu est de 0,25 à 15 % en masse de la composition totale.

9. Produit pour le soin oral selon l'une quelconque des revendications précédentes qui comprend deux phases : la première phase étant non aqueuse de sorte que de l'eau est présente dans une quantité qui n'est pas supérieure à 5 % en masse et la seconde phase étant aqueuse.

10. Produit pour le soin oral selon la revendication 9, dans lequel au moins 95 % en masse de la source de phosphate se trouvent dans la phase aqueuse.

11. Produit pour le soin oral selon l'une quelconque des revendications 9 à 10, dans lequel la source de calcium se trouve dans la phase non aqueuse.

12. Produit pour le soin oral selon l'une quelconque des revendications précédentes qui est substantiellement exempt (moins de 0,05 % en masse du produit total) de peroxyde.

13. Procédé de blanchiment des dents comprenant les étapes suivantes : application aux dents du produit décrit dans l'une quelconque des revendications précédentes.

14. Kit pour éclaircir les dents comprenant :
i) le produit selon l'une quelconque des revendications 1 à 12 ;
et
ii) une source de lumière UV.
